# EUROPEAN PATENT SPECIFICATION

(11) **EP 3 132 042 B1**
(45) Date of publication and mention of the grant of the patent: **20.02.2019**
(21) Application number: 15717878.1
(22) Date of filing: 20.04.2015
(51) Int. Cl.: C12N 15/86, A61K 48/00

(54) **A METHOD OF TREATING PERIPHERAL NEUROPATHIES AND MOTOR NEURON DISEASES**
VERFAHREN ZUR BEHANDLUNG PERIPHERER NEUROPATHIEN UND MOTONEURONERKRANKUNGEN
PROCÉDÉ DE TRAITEMENT DE NEUROPATHIES PÉRIPHÉRIQUES ET SCLÉROSE LATÉRALE AMYOTROPHIQUE

(30) Priority: 18.04.2014 EP 14165329
(43) Date of publication of application: 22.02.2017
(73) Proprietor: GENETHON, 91002 Evry Cedex (FR); Wake Forest University Health Sciences, Winston-Salem, NC 27157 (US)
(72) Inventor: BUJ BELLO, Ana Maria, F-75005 Paris (FR); CHILDERS, Martin, K., Edmonds, Washington 98026 (US)
(74) Representative: Cabinet Laurent & Charras
(86) International application number: PCT/EP2015/058505
(87) International publication number: WO 2015/158924

(56) References cited:
- WO-A1-2005/060746
- WO-A1-2009/043936
- WO-A1-2010/129021
- D M MCCARTY ET AL: "Mannitol-facilitated CNS entry of rAAV2 vector significantly delayed the neurological disease progression in MPS IIIB mice", GENE THERAPY, vol. 16, no. 11, 9 July 2009 (2009-07-09), pages 1340-1352, XP055117436, ISSN: 0969-7128, DOI: 10.1038/gt.2009.85
- BRETT D DUFOUR ET AL: "Intrajugular Vein Delivery of AAV9-RNAi Prevents Neuropathological Changes and Weight Loss in Huntington's Disease Mice", MOLECULAR THERAPY, vol. 22, no. 4, 6 January 2014 (2014-01-06), pages 797-810, XP055198378, ISSN: 1525-0016, DOI: 10.1038/mt.2013.289
- STEVEN J GRAY ET AL: "Preclinical Differences of Intravascular AAV9 Delivery to Neurons and Glia: A Comparative Study of Adult Mice and Nonhuman Primates", MOLECULAR THERAPY, vol. 19, no. 6, 12 April 2011 (2011-04-12) , pages 1058-1069, XP055137448, ISSN: 1525-0016, DOI: 10.1038/mt.2011.72
- CHUNPING QIAO ET AL: "Hydrodynamic Limb Vein Injection of Adeno-Associated Virus Serotype 8 Vector Carrying Canine Myostatin Propeptide Gene into Normal Dogs Enhances Muscle Growth", HUMAN GENE THERAPY, vol. 20, no. 1, 2009, pages 1-10, XP055198430, ISSN: 1043-0342, DOI: 10.1089/hum.2008.135
- MANDEL ET AL: "Recombinant adeno-associated viral vectors as therapeutic agents to treat neurological disorders", MOLECULAR THERAPY, NATURE PUBLISHING GROUP, GB, vol. 13, no. 3, March 2006 (2006-03), pages 463-483, XP005326760, ISSN: 1525-0016, DOI: 10.1016/J.YMTHE.2005.11.009
- AY I ET AL: "VEGF increases blood-brain barrier permeability to Evans blue dye and tetanus toxin fragment C but not adeno-associated virus in ALS mice", BRAIN RESEARCH, ELSEVIER, AMSTERDAM, NL, vol. 1234, 9 October 2008 (2008-10-09), pages 198-205, XP025434456, ISSN: 0006-8993, DOI: 10.1016/J.BRAINRES.2008.07.121 [retrieved on 2008-08-12]
- ARAVIND ASOKAN: "Reengineered AAV vectors: old dog, new tricks", DISCOVERY MEDICINE, vol. 9, no. 48, May 2010 (2010-05), page 399, XP055198456, United States
- KENNETH H WARRINGTON ET AL: "Treatment of human disease by adeno-associated viral gene transfer", HUMAN GENETICS, SPRINGER, BERLIN, DE, vol. 119, no. 6, 13 April 2006 (2006-04-13), pages 571-603, XP019428109, ISSN: 1432-1203, DOI: 10.1007/S00439-006-0165-6
- ADAM K BEVAN ET AL: "Systemic Gene Delivery in Large Species for Targeting Spinal Cord, Brain, and Peripheral Tissues for Pediatric Disorders", MOLECULAR THERAPY, vol. 19, no. 11, 2 August 2011 (2011-08-02), pages 1971-1980, XP055137447, ISSN: 1525-0016, DOI: 10.1038/mt.2011.157

### SEQUENCE LISTING

<110> GENETHON WAKE FOREST UNIVERSITY HEALTH SCIENCES
<120> A METHOD OF TREATING PERIPHERAL NEUROPATHIES AND MOTOR NEURON DISEASES
<130> G143-B-41343 PCT
<150> EP14165329
   <151> 2014-04-18
<160> 6
<170> BiSSAP 1.2
<210> 1
   <211> 23
   <212> DNA
   <213> Artificial Sequence
<220>
   <221> source
   <222> 1..23
   <223> /mol_type="unassigned DNA" /note="MTM1 forward" /organism="Artificial Sequence"
<400> 1
   ataagttttg gacataagtt tgc 23
<210> 2
   <211> 20
   <212> DNA
   <213> Artificial Sequence
<220>
   <221> source
   <222> 1..20
   <223> /mol_type="unassigned DNA" /note="MTM1 reverse" /organism="Artificial Sequence"
<400> 2
   catttgccat acacaatcaa 20
<210> 3
   <211> 19
   <212> DNA
   <213> Artificial Sequence
<220>
   <221> source
   <222> 1..19
   <223> /mol_type="unassigned DNA" /note="MTM1 probe" /organism="Artificial Sequence"
<400> 19
   cgacgctgac cggtctcct 19
<210> 4
   <211> 20
   <212> DNA
   <213> Artificial Sequence
<220>
   <221> source
   <222> 1..20
   <223> /mol_type="unassigned DNA" /note="GLU forward" /organism="Artificial Sequence"
<400> 4
   acgctgattg ctcacaccaa 20
<210> 5
   <211> 22
   <212> DNA
   <213> Artificial Sequence
<220>
   <221> source
   <222> 1..22
   <223> /mol_type="unassigned DNA" /note="GLU reverse" /organism="Artificial Sequence"
<400> 5
   ccccaggtct gcttcatagt tg 22
<210> 6
   <211> 21
   <212> DNA
   <213> Artificial Sequence
<220>
   <221> source
   <222> 1..21
   <223> /mol_type="unassigned DNA" /note="GLU probe" /organism="Artificial Sequence"
<400> 6
   cccggcccgt gacctttgtg a 21

## Description

### BACKGROUND OF THE INVENTION

The peripheral nervous system (PNS) consists of nerves and neurons, including peripheral nerves and neuronal ganglia that are located outside the central nervous system (CNS) or extended outside the CNS from the brain and spinal cord.

PNS is involved in numerous neurological disorders, inherited or acquired, such as Charcot-Marie-Tooth disease, diabetic, infectious, toxic and drug-related, or immune-related neuropathies.

Effective clinical interventions for those diseases are very limited. Gene therapy represents a novel therapeutic strategy for the PNS diseases. However, efficient gene transfer of the PNS remains critical for gene therapy of inherited and acquired peripheral neuropathies.

It has been reported that adeno-associated virus (AAV) vectors can efficiently transduce dorsal root ganglion (DRG) neurons. However, it needs a delicate microneurosurgical technique to deliver AAV to the DRG (Glatzel et al., 2000, Proc. Natl. Acad. Sci. U.S.A. 97, 442-447).

Foust et al. (2008, Hum. Gen. Ther. 19, 61-70) reported that AAV could transduce nerve fibers in the dorsal horn and column, indicating DRG transduction, when AAV serotype 8 vector was systemically delivered into neonatal mice. However, the systemic route is not specific and requires large amounts of therapeutic gene.

Zheng et al. (2010, Hum. Gen. Ther. 21(1), 87-97) reported the capacity of AAV8 in transducing PNS in neonatal mice by intraperitoneal injection and in adult mice by intramuscular injection, in tibialis anterior and gastrocnemius muscles of the hind leg. In both cases, efficient and long-term gene transfer was found in the white matter of the spinal cord, DRG neurons and peripheral nerves. These results support the mechanism of retrograde transport of AAV vectors from muscle to the spinal cord, rather than blood-brain barrier crossing. However, intramuscular delivery requires multiple injection sites associated with a lower efficiency.

Horns et al. (2011, Gene Therapy 18, 622-630) reported the tropism and transduction efficiency of different AAV pseudotypes after sciatic nerve injection. It was observed that AAV8 allows the specific transduction of Schwann cells, offering a gene therapy strategy for peripheral nerve regeneration. However, the local administration in a nerve is not applicable at the clinical level, especially for safety reasons.

Bevan et al. (2011, Mol. Therapy 19(11), 1971-80) reports the efficient delivery by intravenous injection of AAV9 vectors to CNS and peripheral tissues in macaques of any age. This offers a promising therapeutic solution for pediatric disorders such as spinal muscular atrophy (SMA). To reduce peripheral organ toxicity, occlusion of blood flow into liver during intravascular injection was tested. Moreover, it was shown that CSF (cerebrospinal fluid) injection targets motor neurons and restricts gene expression to CSF.

Weismann et al. (2013, Mol. Therapy 21, S147-148) reports that intravenous administration (IV infusion) of AAV9-βgal in a GM1 (gangliosidosis) mouse model expresses enzyme in the CNS and delays disease onset with gender differences.

WO 2010/129021 relates to the use of self-complementary (sc) AAV vectors for treating neurodegenerative disorders, e.g. SMA or ALS (amyotrophic lateral sclerosis). Examples illustrate intracerebroventricular and spinal cord injection.

Wang et al. (2014, Human Mol. Genetics 23(3), 668-81) reports efficient RNAi therapy for ALS by intrathecal injection of AAV (AAVrh10).

Dehay et al. (2012, Scientific Reports 2) reports that systemic (IV) scAAV9 mediates brain transduction in newborn rhesus macaques, as monitored by GFP. Gray et al. (2011, Molecular Therapy 19(6), 1058-1069) compares intravascular AAV9 (ss and sc) delivery to neurons and glia in adult mice and nonhuman primates. WO 2009/043936 discloses the use of double-stranded self-complementary AAV vector for gene delivery to motor neurons, glial cells or spinal cord by peripheral (e.g. IV or IM) administration.

Moreover, recent reviews (Bourdenx et al. 2014, Front Mol Neurosci. 7:50; Murlidharan et al. 2014, Front Mol Neurosci. 7:76; Karda et al. 2014, Front Mol Neurosci. 7:89) list all these options for gene delivery to the central nervous system (CNS) using Adeno-Associated virus. Systemic delivery, especially IV injection, appears promising since it would avoid invasive brain surgery. However, efforts are still required concerning the control of transgene expression, cell specificity and vector optimization.

Mccarty et al. (2009, Gene Therapy 16:11, 1340-52) discloses the use of an AAV2 vector encoding the enzyme NaGlu for treating the neurological disease MPS IIIB. The construct is injected in the tail vein of mice. It is shown that a pre-injection (8 min before) of mannitol (an osmotic agent increasing the BBB permeability) in the same vein increases the CNS transduction.

Dufour et al. (2014, Molecular Therapy 22:4, 797-810) reports the administration in the mouse intrajugular vein of an AAV9-RNAi, said RNAi targeting the mutated protein mHTT responsible for Huntington's disease (HD). In this case, mannitol pretreatment does not increase AAV9 transduction in the CNS.

Gray et al. (2011, Molecular Therapy 19(6), 1058-69) studies the CNS transduction of AAV9 vectors (encoding GFP) after injection in the tail vein of mice, possibly after mannitol injection. It also reports the injection of non-human primates in the saphenous vein or in the carotid artery of a composition further comprising 5% sorbitol.

Qiao et al. (2009, Human Gene Therapy 20(1), 1-10) deals with the treatment of a muscular disease in dogs based on the "hydrodynamic limb vein injection" of an AAV8 vector. This involves applying a pressure via a tourniquet.

Mandel et al. (2006, Molecular Therapy 13(3), 463-83) lists the AAV treatments available for neurological disorders.

Therefore, there is a need in the art for effective, simple and minimally invasive delivery methods to the PNS and/or CNS. The present invention satisfies this unmet need.

### DESCRIPTION OF THE INVENTION

The present invention is based on the observation that, one year after loco-regional infusion of an AAV8 vector in the saphenous vein of a dog, a high amount of said vector was detected in the infused sciatic nerve.

In an unexpected manner, this route of administration which was foreseen for muscular delivery only so far, and not for PNS or CNS disorders, has been shown to be very efficient for delivery to the PNS and/or the CNS.

### Definitions

The articles "a" and "an" are used herein to refer to one or to more than one (*i.e.,* to at least one) of the grammatical object of the article. By way of example, "an element" means one element or more than one element.

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

"Isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*i.e.,* rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

The term "polynucleotide" as used herein is defined as a chain of nucleotides. Furthermore, nucleic acids are polymers of nucleotides. Thus, nucleic acids and polynucleotides as used herein are interchangeable. One skilled in the art has the general knowledge that nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR and the like, and by synthetic means.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

"Homologous" or "identical" refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous or identical at that position. The percent of homology/identity between two sequences is a function of the number of matching or homologous positions shared by the two sequences divided by the number of positions compared X 100. For example, if 6 of 10 of the positions in two sequences are matched or homologous then the two sequences are 60% homologous/identical. Generally, a comparison is made when two sequences are aligned to give maximum homology/identity.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, and the like.

"Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, such as cosmids, plasmids (*e.g.,* naked or contained in liposomes) and viruses (*e.g.,* lentiviruses, retroviruses, adenoviruses, and adeno-associated viruses) that incorporate the recombinant polynucleotide.

The term "promoter" as used herein is defined as a DNA sequence recognized by the synthetic machinery of the cell, or introduced synthetic machinery, required to initiate the specific transcription of a polynucleotide sequence.

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

A "constitutive" promoter is a nucleotide sequence which, when operably linked with a polynucleotide which encodes or specifies a gene product, causes the gene product to be produced in a cell under most or all physiological conditions of the cell.

An "inducible" promoter is a nucleotide sequence which, when operably linked with a polynucleotide which encodes or specifies a gene product, causes the gene product to be produced in a cell substantially only when an inducer which corresponds to the promoter is present in the cell.

A "tissue-specific" promoter is a nucleotide sequence which, when operably linked with a polynucleotide encodes or specified by a gene, causes the gene product to be produced in a cell substantially only if the cell is a cell of the tissue type corresponding to the promoter.

The term "abnormal" when used in the context of organisms, tissues, cells or components thereof, refers to those organisms, tissues, cells or components thereof that differ in at least one observable or detectable characteristic (e.g., age, treatment, time of day, etc.) from those organisms, tissues, cells or components thereof that display the "normal" (expected) respective characteristic. Characteristics which are normal or expected for one cell or tissue type, might be abnormal for a different cell or tissue type.

The terms "patient," "subject," "individual," and the like are used interchangeably herein, and refer to any animal, or cells thereof whether *in vitro* or *in situ,* amenable to the methods described herein. In certain non-limiting embodiments, the patient, subject or individual is a human.

A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

A disease or disorder is "alleviated" if the severity of a symptom of the disease or disorder, the frequency with which such a symptom is experienced by a patient, or both, is reduced. A disease or disorder is "cured" if the severity of a symptom of the disease or disorder, the frequency with which such a symptom is experienced by a patient, or both, is eliminated.

A "therapeutic" treatment is a treatment administered to a subject who exhibits signs of pathology, for the purpose of diminishing or eliminating those signs.

As used herein, "treating a disease or disorder" means reducing the frequency or severity of at least one sign or symptom of a disease or disorder experienced by a subject. Disease and disorder are used interchangeably herein in the context of treatment.

An "effective amount" of a compound is that amount of compound which is sufficient to provide a beneficial effect to the subject to which the compound is administered. The phrase "therapeutically effective amount," as used herein, refers to an amount that is sufficient or effective to prevent or treat (delay or prevent the onset of, prevent the progression of, inhibit, decrease or reverse) a disease or disorder or condition, including alleviating symptoms thereof. An "effective amount" of a delivery vehicle is that amount sufficient to effectively bind or deliver a compound.

### Description

The present application discloses a strategy of delivering a molecule to the peripheral nervous system (PNS) and/or to the central nervous system (CNS) of a subject. By using a marker, e.g. a GFP protein, the methods disclosed herein provide the potential to image and therefore visualize tissues. Alternatively, by delivering a therapeutic molecule to a tissue, a disease or a disorder of the PNS and/or the CNS may be treated.

Whereas efficient delivery to the PNS has been demonstrated in the present application, it is believed that delivery to the CNS would occur via retrograde transport as previously reported (Zheng et al. (2010, Hum. Gen. Ther. 21(1), 87-97). In one embodiment, it is disclosed a method for delivering a molecule to the peripheral nervous system (PNS) and/or to the central nervous system (CNS) of a subject comprising administrating to said subject, by regional or loco-regional infusion, a composition comprising said molecule. In other words, it is disclosed a composition comprising a molecule for use in the delivery of said molecule to the peripheral nervous system (PNS) and/or to the central nervous system (CNS), wherein the composition is administered by regional or loco-regional infusion. Also disclosed is the use of a molecule for the preparation of a diagnostic or therapeutic composition (medicament) for delivering said molecule to PNS and/or CNS by regional or loco-regional infusion.

In a preferred embodiment, the composition comprises an effective amount of the molecule.

A route of administration is the path by which a drug or other substance is taken into the body. Routes of administration are generally classified by the location at which the substance is applied (e.g. oral or intravenous administration). Routes can also be classified based on where the target of action is. Action may be topical (local), enteral (system-wide effect, but delivered through the gastrointestinal (GI) tract), or parenteral (systemic action, but delivered by routes other than the GI tract).

Available modes of parenteral administration include:
- intravenous (IV, into a vein) or intra-arterial (into an artery), generally named "systemic administration";
- intraosseous infusion (into the bone marrow) which is an indirect intravenous access because the bone marrow drains directly into the venous system;
- intra-muscular (IM);
- intracerebral into the brain parenchyma;
- intrathecal into the spinal canal;
- subcutaneous (sc) under the skin.

The term "injection" (or "perfusion" or "infusion") encompasses intravenous (IV), subcutaneous (SC) and intramuscular (IM) administration. Injections are usually performed using syringes or catheters. Injections act rapidly, with onset of action in 15-30 seconds for IV, 10-20 minutes for IM, and 15-30 minutes for SC. Intravascular injections allow ubiquitous distribution in a very short time, with an increased risk of overdose and/or side effects. On the contrary, intramuscular injections allow slow and local diffusion, with the risk of not reaching the target tissue or with an inefficient dose.

According to the invention, the composition is administered to an isolated limb to ensure a local or regional (loco-regional) infusion or perfusion. In other words, it is disclosed the regional delivery of the composition in a leg and/or arm by an intravascular administration, i.e. via a vein (transveneous) or an artery, performed under pressure. This is usually achieved by using a tourniquet to temporarily arrest blood circulation while allowing a regional diffusion of the infused product, as e.g. disclosed by Petrov et al. (2011, Methods Mol Biol 709:277-86), Arruda et al. (2010, Blood 115(23):4678-88) and Zheng Fan et al. (2012, Molecular Therapy 20(2), 456-461).

In comparison with "classical" systemic administration (especially IV), when using such a loco-regional administration, the injected product first diffuses locally at the site of injection and then (when the pressure is released) enters the blood circulation.

Such an *in vivo* method for delivering a polynucleotide to a tissue is generally disclosed in WO 2005/060746 which teaches:
a) inserting a viral vector in a solution into the lumen of a (afferent or efferent) vessel;
b) increasing vessel permeability within the tissue;
c) delivering the viral vector to the tissue outside the vessel.

In practice, increasing the vessel permeability can be achieved by injecting a large volume, injecting the solution rapidly, increasing hydrostatic pressure against the vessel wall (e.g. by obstructing outflow from the blood vessel), increasing osmotic pressure, occluding fluid flow through vessels, and injecting a solution that contains a vasodilator.

This route of administration, usually called "regional (loco-regional) infusion", "administration by isolated limb perfusion" or "high-pressure transvenous limb perfusion" has been successfully used as a gene delivery method in muscular dystrophy (Zheng et al. (2012, Molecular Therapy 20(2), 456-461).

According to one embodiment, the invention relates to a composition comprising an adeno-associated viral (AAV) vector harboring a nucleic acid sequence encoding a diagnostic or a therapeutic molecule for use in the diagnosis or treatment of a disease of the peripheral nervous system (PNS) and/or of the central nervous system (CNS) of a subject, wherein the composition is administered by intravascular route under conditions increasing the vascular permeability, by injecting a large volume, by injecting the composition rapidly, by increasing hydrostatic pressure against the vessel wall and/or by occluding fluid flow through vessel, advantageously by intravascular route under conditions increasing the vascular permeability at the site of administration by increasing hydrostatic pressure against the vessel wall and/or occluding fluid flow through vessel.

In one embodiment, the composition is injected in a limb of the subject. In one embodiment, the subject is a mammal, preferably a dog, a non-human primate or a human. When the subject is a human, the limb can be the arm or the leg. When the subject is an animal, the limb can be the upper limb or the lower limb.

According to one embodiment, the composition is administered in the lower part of the body of the subject, e.g. in the groin or below the knee.

In one embodiment, the composition is administered to a peripheral vein, advantageously the saphenous vein, more advantageously the distal saphenous vein. More generally, the composition can be injected in:
- the leg via: the superficial veins (ie. great and small saphenous); the deep veins (ie. femoral, popliteal, anterior or posterior tibial veins); the arteries (ie. femoral, deep femoral, popliteal, anterior or posterior tibial arteries);
- the arm via: the superficial veins (ie digital, metacarpal, cephalic, basilic, median antibrachial veins); the deep veins (ie. digital, metacarpal, radial, ulnar, brachial veins); the arteries (brachial, radial and ulnar arteries and branches).

According to a preferred embodiment, the composition is administered by intravenous injection. According to another embodiment, the composition is administered in a vessel of a limb of the subject.

According to one embodiment, the volume of the solution is in favor of an increased permeability of vessels. The volume of the composition to be infused can be up to 50% of the limb volume but can be in a range that varies between about 5 and 20% of the limb volume.

The typical dose in dogs or humans can vary between 10 and 20 ml/kg of body weight, and the dose is typically calculated to be 15 ml/kg of body weight.

The composition comprising the molecule of interest is preferably a saline composition, advantageously a Ringer's lactate solution, e.g. 0.9% saline.

According to another embodiment, the rate of solution injection is in favor of an increased permeability of vessels. In one embodiment, the average flow rate is comprised between 50 and 150 ml/min, advantageously between 60 and 80 ml/min.

In one embodiment, the pressure to be applied (tourniquet pressure or maximum line pressure) is below 100 000 Pa, advantageously below 50 000 Pa. In a preferred embodiment, the pressure applied is around 300 torr (40 000 Pa).

In one embodiment, the blood circulation of the limb is stopped using a tourniquet. Advantageously, the tourniquet is placed above the site of injection, e.g. above the elbow or above the knee.

According to another embodiment, the tourniquet is tightened for several minutes, typically between about 1 and 20 minutes, for example about 15 minutes. In a preferred embodiment, the tourniquet is applied before and during the administration, for example about 10 minutes prior to and about 5 minutes during the infusion. More generally, the pressure is applied for several minutes, typically between about 1 and 20 minutes, for example about 15 minutes. In a preferred embodiment, the pressure is applied before and during the administration, for example about 10 minutes prior to and about 5 minutes during the infusion.

According to a particular embodiment, a tourniquet is positioned at the level of the groin and adjusted until the femoral pulse is no longer detectable by ultrasound to transiently block blood inflow to the target limb. A tight extensible wrap is applied in a distal to proximal direction exsanguinated the limb before the tourniquet is tightened. Vector is suspended in Ringer's lactate solution at 20% of the total hind limb volume (determined by water volume displacement) and administered via a 14 gauge catheter placed into a distal branch of the peripheral saphenous vein on the dorsum of the paw. The tourniquet is tightened for a total of 15 minutes (10 minutes prior to and 5 minutes during the infusion).

In the frame of the invention, the peripheral nervous system (PNS) includes the nerves and neurons that are located outside the central nervous system (CNS) or extended outside the CNS from the brain and spinal cord. It includes peripheral nerves (ie. sciatic nerve...), neuronal ganglia (ie. dorsal root ganglia...) and neurons in the spinal cord (motoneurons).

In the frame of the invention, the central nervous system (CNS) includes the spinal cord, in particular motor neurons (or motoneurons).

In an unexpected manner, it was observed that the molecule delivered by the method disclosed therein could be detected in the target tissues 1 year after a single loco-regional infusion. In one embodiment, it is provided a method, wherein the molecule is detected in the PNS and/or the CNS of the subject for 1 day, 3 days, 1 week, 2 weeks, 1 month, 3 months, 6 months, 1 year, 5 years or longer. However, even a transient expression/detection may be useful for imaging of tissues, or for treatment of a subject in need thereof.

In a specific embodiment, the method comprises a single administration of the composition.

The application contemplates delivery of any type or class of molecule using the methods disclosed herein. The molecule may be a chemical molecule, an antibody, a peptide or a protein, a nucleic acid, or a vector, for example a viral vector. The molecule also may be one designed for labeling and imaging, or it may be a therapeutic molecule.

In one embodiment, for labeling or diagnostic purposes, the molecule is any molecule which allows the visualization, advantageously the specific and selective visualization of the target tissues, using the available detection and imaging techniques (radioactivity, fluorescence, MRI, ...). Non-limiting examples of such molecules include, a contrast agent, a fluorophore, or a fluorescently labeled imaging agent.

Of special interest is a therapeutic molecule, able to cure or alleviate a disease or a disorder of the PNS and/or of the CNS. In one embodiment, said disease is associated with one or more defective proteins.

In this context, the therapeutic molecule can be:
- A biologically functional protein or an active fragment thereof. As would be understood in the art, an active fragment is a portion or portions of a full length sequence that retain the biological function of the full length sequence;
- An isolated nucleic acid sequence encoding said protein or fragment, i.e. a transgene, nude or harbored by an expression vector. More generally, it can be an isolated nucleic acid encoding a peptide having substantial homology to the peptides disclosed herein. Preferably, the nucleotide sequence of an isolated nucleic acid encoding a peptide is "substantially identical", that is, is about 60% identical, more preferably about 70% identical, even more preferably about 80% identical, more preferably about 90% identical, even more preferably, about 95% identical, and even more preferably about 99% identical to a nucleotide sequence of an isolated nucleic acid encoding said protein or fragment;
- An isolated nucleic acid sequence that is capable of correcting a defect in a native protein, e.g. an antisense RNA (siRNA, shRNA) inducing exon skipping/inclusion or silencing gene expression, a microRNA (miRNA), other RNA and DNA fragments.

In one embodiment, the isolated nucleic acid sequence encodes a protein where the nucleic acid is referred to as a transgene. In a particular embodiment, said transgene corresponds to an open reading frame and is delivered by the method of the invention, possibly via an expression vector.

In one embodiment, the sequence of the transgene corresponds to a native (endogenous) sequence present in the subject. In a specific embodiment, the endogenous sequence is defective, i.e. displays one or more mutations leading to the lack of the corresponding protein or the production of a partially or fully inactive protein, or to the corresponding protein with a gain-of-function, notably in the PNS and/or CNS, and is associated with a disease.

More generally, the molecule of interest can be a therapeutic protein or a sequence encoding said protein as disclosed above.

Other molecules of interest include neurotrophic factors (NGF, BDNF, NT3, CNTF, GDNF, neurturin, persephin, artemin, ...), trophic factors (IGF1, IGF2, ...), apoptosis or cell death-inducing genes (e.g. caspases).

The nucleic acid sequence can be single- or double-stranded DNA, RNA or cDNA.

In one embodiment, the nucleic acid is administered as a naked nucleic sequence. In order to facilitate the cell transduction, the nucleic acid sequence can be associated with various structures such as, for example, systems for colloidal dispersions (nanocapsules, microspheres, ...) or lipid-based systems (emulsions, micelles, liposomes, ...).

According to the invention, the composition comprises a plasmid or a vector. According to a specific embodiment, the isolated nucleic acid is inserted into the vector. In brief summary, the expression of natural or synthetic nucleic acids is typically achieved by operably linking a nucleic acid or portions thereof to a promoter, and incorporating the construct into an expression vector. The vectors to be used are suitable for replication and, optionally, integration in eukaryotic cells. Typical vectors contain transcription and translation terminators, initiation sequences, and promoters useful for regulation of the expression of the desired nucleic acid sequence.

According to the invention, the composition comprises an expression vector, advantageously a viral vector. In one embodiment, the viral vector is selected from the group consisting of a baculoviral vector, herpes viral vector, lentiviral vector, retroviral vector, adenoviral vector, and adeno-associated viral (AAV) vector. According to the invention the composition comprises an AAV vector, advantageously an AAV8 vector.

According to the invention, the composition comprises an AAV vector as a vehicle for delivery of the molecule of interest (diagnostic or therapeutic).

In the context of a loco-regional administration, the dose injected may vary between 10¹² and 10¹⁵ vg/kg of the patient body, preferably between 10¹³ and 10¹⁴ vg/kg; e.g. 2.5 ou 5.10¹³ vg/kg.

Adeno-associated viral (AAV) vectors have become powerful gene delivery tools for the treatment of various disorders. AAV vectors possess a number of features that render them ideally suited for gene therapy, including a lack of pathogenicity, minimal immunogenicity, and the ability to transduce postmitotic cells in a stable and efficient manner. Expression of a particular gene contained within an AAV vector can be specifically targeted to one or more types of cells by choosing the appropriate combination of AAV serotype, promoter, and delivery method.

In one embodiment, the nucleic acid sequence is contained within an AAV vector. More than 100 naturally occurring serotypes of AAV are known. Many natural variants in the AAV capsid exist, allowing identification and use of an AAV with properties specifically suited for PNS and/or CNS. AAV viruses may be engineered using conventional molecular biology techniques, making it possible to optimize these particles for cell specific delivery of nucleic acid sequences, for minimizing immunogenicity, for tuning stability and particle lifetime, for efficient degradation, for accurate delivery to the nucleus.

As mentioned above, the use of AAVs is a common mode of exogenous delivery of DNA as it is relatively non-toxic, provides efficient gene transfer, and can be easily optimized for specific purposes. Among the serotypes of AAVs isolated from human or non-human primates (NHP) and well characterized, human serotype 2 is the first AAV that was developed as a gene transfer vector. Other currently used AAV serotypes include AAV1, AAV3, AAV4, AAV5, AAV6, AAV7, AAV8, AAV9, AAV10, AAV11 and AAV12. In addition, non-natural engineered variants and chimeric AAV can also be useful.

Desirable AAV fragments for assembly into vectors include the cap proteins, including the vp1, vp2, vp3 and hypervariable regions, the rep proteins, including rep 78, rep 68, rep 52, and rep 40, and the sequences encoding these proteins. These fragments may be readily utilized in a variety of vector systems and host cells.

Such fragments may be used alone, in combination with other AAV serotype sequences or fragments, or in combination with elements from other AAV or non-AAV viral sequences. As used herein, artificial AAV serotypes include, without limitation, AAV with a non-naturally occurring capsid protein. Such an artificial capsid may be generated by any suitable technique, using a selected AAV sequence (e.g., a fragment of a vp1 capsid protein) in combination with heterologous sequences which may be obtained from a different selected AAV serotype, non-contiguous portions of the same AAV serotype, from a non-AAV viral source, or from a non-viral source. An artificial AAV serotype may be, without limitation, a chimeric AAV capsid, a recombinant AAV capsid, or a "humanized" AAV capsid. Thus exemplary AAVs, or artificial AAVs, include AAV2/8 (US 7,282,199), AAV2/5 (available from the National Institutes of Health), AAV2/9 (WO2005/033321), AAV2/6 (US 6,156,303), and AAVrh8 (WO2003/042397), among others. In one embodiment, the vectors useful in the compositions and methods described herein contain, at a minimum, sequences encoding a selected AAV serotype capsid, e.g., an AAV8 capsid, or a fragment thereof. In another embodiment, useful vectors contain, at a minimum, sequences encoding a selected AAV serotype rep protein, e.g., AAV8 rep protein, or a fragment thereof. Optionally, such vectors may contain both AAV cap and rep proteins. In vectors in which both AAV rep and cap are provided, the AAV rep and AAV cap sequences can both be of one serotype origin, e.g., all AAV8 origin. Alternatively, vectors may be used in which the rep sequences are from an AAV serotype which differs from that which is providing the cap sequences. In one embodiment, the rep and cap sequences are expressed from separate sources (e.g., separate vectors, or a host cell and a vector). In another embodiment, these rep sequences are fused in frame to cap sequences of a different AAV serotype to form a chimeric AAV vector, such as AAV2/8 (US 7,282,199).

In the AAV vectors used in the present invention, the AAV genome may be either a single stranded (ss) nucleic acid or a double stranded (ds), self complementary (sc) nucleic acid.

According to the invention, the molecule of interest is a nucleic acid sequence as defined above.

Advantageously, the nucleic acid sequence of interest is inserted between the ITR (« Inverted Terminal Repeat ») sequences of the AAV vector.

As known in the art, recombinant viral particles can be obtained, e.g. by tri-transfection of 293 HEK cells, by the herpes simplex virus system and by the baculovirus system. The vector titers are usually expressed as viral genomes per ml (vg/ml).

In one embodiment, the expression vector comprises regulatory sequences, especially a promoter sequence. Such promoters can be natural or synthetic (artificial) promoters, inducible or constitutive.

In one embodiment, the promoter is an ubiquitous promoter or having a low tissue-specificity. As an example, the expression vector can harbor the phosphoglycerate kinase 1 (PGK), EF1, β-actin, CMV promoter.

In a preferred embodiment, the promoter sequence is chosen in order to adequately govern the expression of the nucleic acid sequence placed under its control, in terms of expression level but also of tissue specificity. In one embodiment, the expression vector comprises a PNS and/or CNS specific promoter, such as the P0, NSE, SYN1, Hb9 or Thy-1 promoter.

A non-exhaustive list of other possible regulatory sequences is:
- a polyadenylation signal, e.g. the polyA of the gene of interest, the polyA of SV40 or of beta hemoglobin (HBB2), advantageously in 3' of the sequence of interest ;
- sequences for transcript stabilization, e.g. intron 1 of hemoglobin (HBB2);
- enhancer sequences ;
- miRNAs target sequences.

To date, more than 40 genes have been involved in Charcot-Marie-Tooth neuropathies, and more than 15 genes in diseases affecting motoneurons. Among the proteins of interest which defect is involved in a disease of the PNS and/or CNS are:
- Charcot-Marie-Tooth neuropathies: *PMP22, GJB1, MPZ, LITAF, EGR2, NEFL, GAN1, KIF1B, MFN2, TRPV4, GDAP1, DYNC1H1, RSAM1, GNB4, HSPB1, HSPB3, HSPB8, GARS, YARS, AARS, HARS, KARS, MTMR2, MTMR13, RAB7, SPTLC1, SPTLC2, DNM2, PDK3, SH3TC2, NDRG1, PRX, HK1, FGD4, FIG4, CTDP1, LMNA, MED25, PRPS1, FBLN5, INF2, BSCL2, DCTN1, SLC5A7, SETX, REEP1, IGHMPB2, ATP7A;*
- Motoneuron diseases: *SMN1, SOD1, TARDBP, FUS, C9ORF72, SETX, VAPB. ANG, FIG 4, OPTN, VCP, alsin, spatacsin, UBQLN2, SIGMAR1, DCTN1.*

As an example, the myotubularin (MTM1) gene family comprises 15 members, and mutations in two members (MTMR2, MTMR13) are associated with diseases of the PNS. In this context, the delivery of the functional, possibly native protein, or of the corresponding gene should treat these diseases or even cure them.

According to another aspect, it is provided a method for treating a disease of the peripheral nervous system (PNS) and/or of the central nervous system (CNS) in a subject comprising administrating to said subject, by regional or loco-regional infusion as defined above, a composition comprising a therapeutic molecule. In other words, it is disclosed a composition comprising a therapeutic molecule for use in treating a disease of the peripheral nervous system (PNS) and/or of the central nervous system (CNS), wherein the composition is administered by regional or loco-regional infusion as defined above. Also disclosed is the use of a molecule for the preparation of a medicament for treating a disease of the peripheral nervous system (PNS) and/or of the central nervous system (CNS), wherein the medicament is administered by regional or loco-regional infusion as defined above.

In a preferred embodiment, the composition comprises a therapeutically effective amount of the molecule.

Of specific interest are the peripheral neuropathies and the motor neuron diseases. Inherited as well as acquired diseases are concerned. Examples of inherited peripheral neuropathies are Charcot-Marie-Tooth (CMT) neuropathies. Examples of neuromuscular disorders with motoneuron (CNS) involvement are spinal muscular atrophy (SMA) and amyotrophic lateral sclerosis (ALS).

Charcot-Marie-Tooth neuropathies include: demyelinating CMT (AD-CMT1 and CMT4 forms), axonal CMT (AD-CMT2 and AR-CMT2 forms), intermediate CMT (DI-CMT forms), X-linked CMT (D-CMTX and R-CMTX forms), CMT 'plus', dHMN (AD-HMN, R-HMN, X-HMN forms). In this list, A means "autosomal", X means "X-linked", R means "recessive", D means "dominant".

As disclosed therein, the composition comprises at least one active molecule, possibly different molecules. Such a composition can also include a pharmaceutically acceptable inert vehicle. Various excipients, stabilizers and other suitable compounds known to those skilled in the art can be envisaged in such a composition.

Since the composition is to be administered by loco-regional infusion, it will preferably be in liquid form. Determining the vector concentration, the amount to be injected and the frequency of injections is part of normal practice for those skilled in the art.

According to another aspect, it is disclosed a kit comprising a composition as defined above and any piece of material dedicated to the regional or loco-regional infusion, advantageously a tourniquet system and/or a device for monitoring the pressure applied at the site of injection or the pulse of the infused vein or artery (e.g. ultrasound device). Such a kit can also comprise an injector such as a syringe, a needle and/or a catheter.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Oligonucleotide Synthesis" (Gait, 1984); "Culture of Animal Cells" (Freshney, 2010); "Methods in Enzymology" "Handbook of Experimental Immunology" (Weir, 1997); "Gene Transfer Vectors for Mammalian Cells" (Miller and Calos, 1987); "Short Protocols in Molecular Biology" (Ausubel, 2002); "Polymerase Chain Reaction: Principles, Applications and Troubleshooting", (Babar, 2011); "Current Protocols in Immunology" (Coligan, 2002). These techniques are applicable to the production of the polynucleotides and polypeptides of the invention, and, as such, may be considered in making and practicing the invention. Particularly useful techniques for particular embodiments will be discussed in the sections that follow.

It is to be understood that wherever values and ranges are provided herein, all values and ranges encompassed by these values and ranges, are meant to be encompassed within the scope of the present invention. Moreover, all values that fall within these ranges, as well as the upper or lower limits of a range of values, are also contemplated by the present application.

The following examples further illustrate aspects of the present invention. However, they are in no way a limitation of the teachings or disclosure of the present invention as set forth herein.

### EXPERIMENTAL EXAMPLES

The invention is further described in detail by reference to the following experimental examples. These examples are provided for purposes of illustration only, and are not intended to be limiting unless otherwise specified.

Without further description, it is believed that one of ordinary skill in the art can, using the preceding description and the following illustrative examples, make and utilize the compositions of the present invention and practice the claimed methods. The following working examples therefore, specifically point out the preferred embodiments of the present invention, and are not to be construed as limiting in any way the remainder of the disclosure.

### Materials and Methods:

### Animals

XLMTM dogs were described previously (Beggs et al., 2010, Proc Natl Acad Sci USA 107(33):14697-702). Affected males were identified by polymerase chain reaction-based genotyping, as described.

### Preparation and administration of rAAV8-MTM1 in dogs

The recombinant adeno-associated virus vector containing a canine myotubularin cDNA regulated by the desmin promoter, rAAV2/8-pDesmin-*MTM1* canine (designated rAAV8-*MTM1*), was produced in a baculovirus/Sf9 system. Two baculovirus batches were generated, one expressing *rep* and *cap* AAV genes and the second bearing the canine *MTM1* cDNA (XM850116, NCBI) downstream from the human desmin promoter (pDesmin). The rAAV*-MTM1* vector particles were produced after baculoviral double infection of insect Sf9 cells and purified from total cell culture using AVB affinity chromatography column (GE Healthcare, AVB Sepharose high performance). The concentration in vg/mL was determined from DNase-resistant particles, as described above. Other routine quality control assays for rAAV vectors were performed, including sterility and purity tests (Yuasa et al., 2007, Gene Ther 14(17):1249-60).

### Intravenous regional limb infusions

In an anesthetized XLMTM dog, the vector rAAV8*-MTM1* (2.5 x 10¹³ vg/kg) diluted in phosphate buffered saline (PBS) was infused into the distal saphenous vein under pressure (300 torr) against a tourniquet as described (Petrov et al., 2011, Methods Mol Biol 709:277-86; Arruda et al., 2010, Blood 115(23):4678-88). Briefly, a tourniquet was positioned at the level of the groin and adjusted until the femoral pulse was no longer detectable by ultrasound to transiently block blood inflow to the target limb. A tight extensible wrap applied in a distal to proximal direction exsanguinated the limb before the tourniquet was tightened. Vector was suspended in PBS at 20% of the total hind limb volume (determined by water volume displacement) and administered via a 14 gauge catheter placed into a distal branch of the peripheral saphenous vein on the dorsum of the paw. The tourniquet was tightened for a total of 15 minutes (10 minutes prior to and 5 minutes during the infusion). One hind limb was infused with vector whereas the contralateral hind limb was not infused.

### Quantification of the viral titers in the dog tissues

The number of vector genomes (vg) per diploid genome (dg) was quantified from 80 ng of total DNA by Taqman real-time PCR using a 7900 HT thermocycler (Applied Biosystem, France). The canine β-glucuronidase gene was used for standardization. Primers used for vector genome (*MTM1*) amplification were:
*5'-ATAAGTTTTGGACATAAGTTTGC-3'* (forward; SEQ ID NO: 1),
*5'-CATTTGCCATACACAATCAA-3'* (reverse; SEQ ID NO: 2); and
*5'-CGACGCTGACCGGTCTCCT-3'* (probe; SEQ ID NO: 3).
Primers and probe used for β-glucuronidase amplification were:
*5'-ACGCTGATTGCTCACACCAA-3'* (forward; SEQ ID NO: 4),
*5'-CCCCAGGTCTGCTTCATAGTTG-3'* (reverse; SEQ ID NO: 5); and
*5'-CCCGGCCCGTGACCTTTGTGA-3'* (probe; SEQ ID NO: 6) (Applied Biosystem).

### Results:

### Locoregional infusion of an AAV8 vector leads to increased transduction in peripheral nerves

A tourniquet was placed on the upper part of the left hind limb of a 9 week-old XLMTM dog and a rAAV8-Des-cMTM1 vector (2.5x10¹³vg/kg) was injected under pressure via the saphenous vein.

One year after vector administration, the dog was euthanized and a large panel of tissues and organs were collected for vector biodistribution analysis.

Quantification of vector genome copies revealed:
- 16 copies in the sciatic nerve of the infused hind limb;
- 1.8 copies in the contralateral sciatic nerve of the non-infused hind limb.

More generally, these experiments revealed that the infused sciatic nerve was the best transduced tissue of the body, with about ∼15 times more vector DNA (16 vg/dg) than in the contralateral nerve (1.8 vg/dg).

### Comparison with IV (intravenous) injection

In parallel experiments, 2 dogs were systemically administered via injection in the saphenous vein with the same quantity of rAAV8-Des-cMTM1 vector (2.5x10¹³vg/kg). Only 1.8 to 5 vector copies were detected in the sciatic nerves.

These data show the superiority of the locoregional infusion versus the systemic administration to deliver recombinant AAV vectors to the peripheral nervous system (PNS), especially to the sciatic nerves. It is believed that CNS delivery would occur via retrograde transport through PNS.

## Claims

1. A composition comprising an adeno-associated viral (AAV) vector harboring a nucleic acid sequence encoding a diagnostic or a therapeutic molecule for use in the in vivo diagnosis or treatment of a disease of the peripheral nervous system (PNS) and/or of the central nervous system (CNS) of a subject, wherein the composition is administered by intravascular route under conditions increasing the vascular permeability by injecting a large volume, by injecting the composition rapidly, by increasing hydrostatic pressure against the vessel wall and/or by occluding fluid flow through vessel.

2. A composition for its use according to claim 1, wherein the composition is administered by intravascular route under conditions increasing the vascular permeability at the site of administration by increasing hydrostatic pressure against the vessel wall and/or occluding fluid flow through vessels.

3. A composition for its use according to claim 1 or 2, wherein the composition is administered by intravascular route under pressure.

4. A composition for its use according to claim 3, wherein the pressure is applied using a tourniquet.

5. A composition for its use according to any of the preceding claims, wherein the composition is administered by intravenous injection.

6. A composition for its use according to any of the preceding claims, wherein the composition is administered in a vessel of a limb of the subject.

7. A composition for its use according to any of the preceding claims, wherein the disease is a peripheral neuropathy or a motor neuron disease.

8. A composition for its use according to claim 7, wherein the disease is selected in the group consisting of: Charcot-Marie-Tooth (CMT) neuropathies, spinal muscular atrophy (SMA), amyotrophic lateral sclerosis (ALS), demyelinating CMT (AD-CMT1 and CMT4 forms), axonal CMT (AD-CMT2 and AR-CMT2 forms), intermediate CMT (DI-CMT forms), X-linked CMT (D-CMTX and R-CMTX forms), CMT 'plus', dHMN (AD-HMN, R-HMN, X-HMN forms).

9. A composition for its use according to any of the preceding claims, wherein the nucleic acid sequence encodes a therapeutic protein involved in diseases of the PNS and/or the CNS, or an active fragment thereof.

10. A composition for its use according to claim 9, wherein the nucleic acid sequence encodes a protein selected in the group consisting of: *PMP22, GJB1, MPZ, LITAF, EGR2, NEFL, GAN1, KIF1B, MFN2, TRPV4, GDAP1, DYNC1H1, RSAM1, GNB4, HSPB1, HSPB3, HSPB8, GARS, YARS, AARS, HARS, KARS, MTMR2, MTMR13, RAB7, SPTLC1, SPTLC2, DNM2, PDK3, SH3TC2, NDRG1, PRX, HK1, FGD4, FIG4, CTDP1, LMNA, MED25, PRPS1, FBLN5, INF2, BSCL2, DCTN1, SLC5A7, SETX, REEP1, IGHMPB2, ATP7A, SMN1, SOD1, TARDBP, FUS, C9ORF72, SETX, VAPB, ANG, FIG 4, OPTN, VCP, alsin, spatacsin, UBQLN2, SIGMAR1, DCTN1,* the myotubularin (MTM1) family, especially MTMR2 and MTMR13.

11. A composition for its use according to any of the preceding claims, wherein the AAV vector is an AAV8 vector.

12. A composition for its use according to any of the preceding claims, wherein the subject is a mammal, advantageously a dog or a human.

13. A composition for its use according to any of the preceding claims, comprising a single administration of the composition.

## Patentansprüche

1. Zusammensetzung, umfassend einen adeno-assoziierten viralen (AAV) Vektor, der eine Nukleinsäuresequenz beherbergt, die ein diagnostisches oder ein therapeutisches Molekül kodiert, zur Verwendung bei der in vivo-Diagnose oder -Behandlung einer Erkrankung des peripheren Nervensystems (PNS) und/oder des zentralen Nervensystems (ZNS) eines Subjekts, worin die Zusammensetzung auf intravaskulärem Weg unter Bedingungen verabreicht wird, die die Gefäßpermeabilität erhöhen, indem ein großes Volumen injiziert wird, indem die Zusammensetzung schnell injiziert wird, indem der hydrostatische Druck gegen die Gefäßwand erhöht wird und/oder indem der Fluidstrom durch das Gefäß verschlossen wird.

2. Zusammensetzung zur Verwendung nach Anspruch 1, worin die Zusammensetzung auf intravaskulärem Weg unter Bedingungen verabreicht wird, die die Gefäßpermeabilität an der Verabreichungsstelle erhöhen, indem der hydrostatische Druck gegen die Gefäßwand erhöht wird und/oder der Fluidstrom durch das Gefäß verschlossen wird.

3. Zusammensetzung zur Verwendung nach Anspruch 1 oder 2, worin die Zusammensetzung auf intravaskulärem Weg unter Druck verabreicht wird.

4. Zusammensetzung zur Verwendung nach Anspruch 3, worin der Druck unter Verwendung eines Tourniquets aufgebracht wird.

5. Zusammensetzung zur Verwendung nach einem der vorstehenden Ansprüche, worin die Zusammensetzung durch intravenöse Injektion verabreicht wird.

6. Zusammensetzung zur Verwendung nach einem der vorstehenden Ansprüche, worin die Zusammensetzung in ein Gefäß einer Gliedmaße des Subjekts verabreicht wird.

7. Zusammensetzung zur Verwendung nach einem der vorstehenden Ansprüche, worin die Krankheit eine periphere Neuropathie oder eine Motoneuronenkrankheit ist.

8. Zusammensetzung zur Verwendung nach Anspruch 7, worin die Krankheit aus der Gruppe ausgewählt ist, die aus dem Charcot-Marie-Tooth (CMT) Syndrom, der spinalen progredienten Muskelatrophie (SMA), der amyotrophen Lateralsklerose (ALS), dem demyelinisierenden CMT-Syndrom (AD CMT1- und CMT4-Formen), dem axonalen CMT-Syndrom (AD-CMT2- und AR-CMT2-Formen), dem intermediäre CMT-Syndrom (DI-CMT Formen), dem X-verknüpfte CMT-Syndrom (D-CMTX- und R-CMTX-Formen), CMT 'plus', dHMN (AD-HMN-, R-HMN-, X-HMN-Formen) besteht.

9. Zusammensetzung zur Verwendung nach einem der vorstehenden Ansprüche, worin die Nukleinsäuresequenz ein therapeutisches Protein, das in Krankheiten des PNS und/oder des ZNS beteiligt ist, oder ein aktives Fragment davon kodiert.

10. Zusammensetzung zur Verwendung nach Anspruch 9, worin die Nukleinsäuresequenz ein Protein kodiert, das aus der Gruppe ausgewählt ist, die aus *PMP22, GJB1, MPZ, LITAF, EGR2, NEFL, GAN1, KIF1B, MFN2, TRPV4, GDAP1, DYNC1H1, RSAM1, GNB4, HSPB1, HSPB3, HSPB8, GARS, YARS, AARS, HARS, KARS, MTMR2, MTMR13, RAB7, SPTLC1, SPTLC2, DNM2, PDK3, SH3TC2, NDRG1, PRX, HK1, FGD4, FIG4, CTDP1, LMNA, MED25, PRPS1, FBLN5, INF2, BSCL2, DCTN1, SLC5A7, SETX, REEP1, IGHMPB2, ATP7A, SMN1, SOD1, TARDBP, FUS, C9ORF72, SETX, VAPB, ANG, FIG 4, OPTN, VCP, Alsin, Spatacsin, UBQLN2, SIGMAR1, DCTN1,* der Myotubularin-(MTM1)-Familie, insbesondere MTMR2 und MTMR13 besteht.

11. Zusammensetzung zur Verwendung nach einem der vorstehenden Ansprüche, worin der AAV-Vektor ein AAV8-Vektor ist.

12. Zusammensetzung zur Verwendung nach einem der vorstehenden Ansprüche, worin das Subjekt ein Säugetier, vorzugsweise ein Hund oder ein Mensch ist.

13. Zusammensetzung zur Verwendung nach einem der vorstehenden Ansprüche, umfassend eine einzige Verabreichung der Zusammensetzung.

## Revendications

1. Composition comprenant un vecteur viral adéno-associé (AAV) contenant une séquence d'acides nucléiques codant une molécule diagnostique ou thérapeutique pour utilisation pour le diagnostic ou le traitement *in vivo* d'une pathologie du système nerveux périphérique (SNP) et/ou du système nerveux central (SNC) d'un sujet, dans laquelle la composition est administrée par voie intravasculaire dans des conditions augmentant la perméabilité vasculaire en injectant un large volume, en injectant la composition rapidement, en augmentant la pression hydrostatique contre la paroi vasculaire et/ou en obstruant le flux de fluide à travers le vaisseau.

2. Composition pour son utilisation selon la revendication 1, dans laquelle la composition est administrée par voie intravasculaire dans des conditions augmentant la perméabilité vasculaire au niveau du site d'administration en augmentant la pression hydrostatique contre la paroi vasculaire et/ou en obstruant le flux de fluide à travers les vaisseaux.

3. Composition pour son utilisation selon la revendication 1 ou 2, dans laquelle la composition est administrée par voie intravasculaire sous pression.

4. Composition pour son utilisation selon la revendication 3, dans laquelle la pression est exercée en utilisant un tourniquet.

5. Composition pour son utilisation selon l'une des revendications précédentes, dans laquelle la composition est administrée par injection intraveineuse.

6. Composition pour son utilisation selon l'une des revendications précédentes, dans laquelle la composition est administrée dans un vaisseau d'un membre du sujet.

7. Composition pour son utilisation selon l'une des revendications précédentes, dans laquelle la pathologie est une neuropathie périphérique ou une maladie des neurones moteurs.

8. Composition pour son utilisation selon la revendication 7, dans laquelle la pathologie est sélectionnée dans le groupe constitué de : les neuropathies de type Charcot-Marie-Tooth (CMT), l'amyotrophie spinale (AMS), la sclérose latérale amyotrophique (SLA), la CMT démyélinisante (formes AD-CMT1 et CMT4), la CMT axonale (formes AD-CMT2 et AR-CMT2), la CMT intermédiaire (formes DI-CMT), la CMT liée au chromosome X (formes D-CMTX et R-CMTX), la CMT « plus », la dHMN (formes AD-HMN, R-HMN, X-HMN).

9. Composition pour son utilisation selon l'une des revendications précédentes, dans laquelle la séquence d'acides nucléiques code une protéine thérapeutique impliquée dans des pathologies du SNP et/ou SNC, ou un de ses fragments actifs.

10. Composition pour son utilisation selon la revendication 9, dans laquelle la séquence d'acides nucléiques code une protéine choisie dans le groupe constitué de : *PMP22, GJB1, MPZ, LITAF, EGR2, NEFL, GAN1, KIF1B, MFN2, TRPV4, GDAP1, DYNC1H1, RSAM1, GNB4, HSPB1, HSPB3, HSPB8, GARS, YARS, AARS, HARS, KARS, MTMR2, MTMR13, RAB7, SPTLC1, SPTLC2, DNM2, PDK3, SH3TC2, NDRG1, PRX, HK1, FGD4, FIG4, CTDP1, LMNA, MED25, PRPS1, FBLN5, INF2, BSCL2, DCTN1, SLC5A7, SETX, REEP1, IGHMPB2, ATP7A, SMN1, SOD1, TARDBP, FUS, C9ORF72, SETX, VAPB, ANG, FIG 4, OPTN, VCP, alsine, spatacsine, UBQLN2, SIGMAR1, DCTN1,* la famille des myotubularines (MTM1), de préférence MTMR2 et MTMR13.

11. Composition pour son utilisation selon l'une des revendications précédentes, dans laquelle le vecteur AAV est un vecteur AAV8.

12. Composition pour son utilisation selon l'une des revendications précédentes, dans laquelle le sujet est un mammifère, avantageusement un chien ou un humain.

13. Composition pour son utilisation selon l'une des revendications précédentes, comprenant une administration unique de la composition.
